# EUROPEAN PATENT SPECIFICATION

(11) **EP 2 814 412 B1**
(45) Date of publication and mention of the grant of the patent: **07.08.2019**
(21) Application number: 13749333.4
(22) Date of filing: 04.02.2013
(51) Int. Cl.: A61B 17/86, A61B 17/70

(54) **BONE FASTENER**
KNOCHENFIXIERVORRICHTUNG
ÉLÉMENT DE FIXATION D'OS

(30) Priority: 13.02.2012 US 201213372144
(43) Date of publication of application: 24.12.2014
(73) Proprietor: Warsaw Orthopedic, Inc., Warsaw, IN 46581 (US)
(72) Inventor: REZACH, William Alan, Atoka, Tennessee 38004 (US)
(74) Representative: Viering, Jentschura & Partner mbB Patent- und Rechtsanwälte
(86) International application number: PCT/US2013/024583
(87) International publication number: WO 2013/122762

(56) References cited:
- WO-A2-2008/089096
- US-A1- 2006 036 252
- US-A1- 2009 062 914
- US-A1- 2009 105 769
- US-A1- 2010 125 302
- US-A1- 2010 298 891
- US-A1- 2011 106 173
- US-A1- 2011 106 174
- US-A1- 2012 016 425

## Description

The present disclosure generally relates to medical devices for the treatment of spinal disorders, and more particularly to a spinal implant system including a bone fastener that provides stabilization while reducing stress on spinal elements.

### BACKGROUND

Spinal disorders such as degenerative disc disease, disc herniation, osteoporosis, spondylolisthesis, stenosis, scoliosis and other curvature abnormalities, kyphosis, tumor, and fracture may result from factors including trauma, disease and degenerative conditions caused by injury and aging. Spinal disorders typically result in symptoms including pain, nerve damage, and partial or complete loss of mobility.

Non-surgical treatments, such as medication, rehabilitation and exercise can be effective, however, may fail to relieve the symptoms associated with these disorders. Surgical treatment of these spinal disorders includes discectomy, laminectomy, fusion and implantable prosthetics. During surgical treatment, one or more rods may be attached via fasteners to the exterior of two or more vertebral members to provide stability to a treated region. This disclosure describes an improvement over these prior art technologies.

Bone fasteners are known from US 2011/106174 A1, WO 2008/089096 A2, and US 2012/016425 A1.

### SUMMARY

According to the present invention, a bone fastener according to claim 1 is provided. Examples of bone fasteners are defined in the dependent claims.

The methods and procedures that do not fall within the scope of the claims do not form part of the invention but represent background art that is useful in understanding the invention.

According to an exemplary embodiment, a spinal implant system is provided. In one embodiment, in accordance with the principles of the present disclosure, the spinal implant system includes a bone fastener. The bone fastener comprises a proximal portion including an inner surface and an outer surface having an extension that includes an inner surface defining at least one projection. A pivoting member is moveable relative to the inner surface of the proximal portion. The pivoting member includes a first surface that defines an implant cavity with the inner surface of the proximal portion and a second surface. A component is engageable with the proximal portion and includes an inner surface that defines an inner cavity configured for disposal of the extension. The component further includes at least one planar surface. A distal portion defines a longitudinal axis and has a first end and a second end configured to penetrate tissue. The first end includes at least one planar surface configured to engage the at least one planar surface of the component. The proximal portion is rotatable relative to the distal portion in a first plane of a body and the pivoting member is rotatable relative to the proximal portion in a second plane of the body.

In one embodiment, the spinal implant system includes at least one bone fastener comprising a receiver defining a first longitudinal axis and including spaced apart arms that include an inner surface of the receiver. At least a portion of the inner surface is threaded and engageable with a setscrew. The receiver includes a distal collar having an inner surface that defines a first lateral opening and a second lateral opening spaced apart from the first lateral opening. The inner surface of the collar includes a first circumferential rim and a second circumferential rim spaced apart from the first circumferential rim. A saddle extends between a first end and a second end. The saddle includes a through opening and defines a first surface configured for slidable engagement with the inner surface of the receiver along an arcuate path. The saddle defines a second concave surface that defines an implant cavity with the receiver. The implant cavity defines a second axis transverse to the first longitudinal axis and is configured for disposal of an implant. A cap is configured for fixed engagement with the distal collar. The cap includes an inner surface that defines an inner cavity configured for disposal of the collar. The cap includes a first inwardly oriented tab that includes a first planar surface and a second inwardly oriented tab that includes a second planar surface. A tissue penetrating shaft extends between a first end and a second end defining a third longitudinal axis. The first end of the shaft includes a head that is engageable with the tabs in a configuration to retain the shaft with the receiver and defines a tool socket configured for alignment with the through opening. The head includes a first planar surface oriented in a first direction to engage the first planar surface of the first tab, a second planar surface oriented in a second direction to engage the second planar surface of the second tab and an arcuate surface disposed therebetween. The planar surfaces are engageable in a configuration to prevent rotation of the shaft relative to the receiver and the cap about the third longitudinal axis. The arcuate surface is engageable and moveable relative to the receiver. The system includes a vertebral rod. The bone fastener is movable between a first configuration such that the receiver is selectively rotatable relative to the shaft in a transverse plane of a body and the saddle is selectively rotatable relative to the receiver in a sagittal plane of the body between a first movable limit defined by engagement of the first end of the saddle with the arcuate surface and a second movable limit defined by engagement of the second end of the saddle with the arcuate surface, and a second configuration such that the setscrew applies a force to the rod disposed in the implant cavity and the rod engages the concave surface of the saddle to fix the bone fastener in an orientation.

A method for treating a spine disorder that is not covered by the present invention comprises the steps of providing a bone fastener comprising: a proximal portion including an inner surface and an outer surface having an extension that includes an inner surface defining at least one projection, a pivoting member being moveable relative to the inner surface of the proximal portion, the pivoting member including a first surface that defines an implant cavity with the inner surface of the proximal portion and a second surface, a component engageable with the proximal portion and including an inner surface that defines an inner cavity configured for disposal of the extension, the component further including at least one planar surface, and a distal portion defining a longitudinal axis and having a first end and a second end configured to penetrate tissue, the first end including at least one planar surface configured to engage the at least one planar surface of the component; attaching the distal portion with vertebrae; providing a vertebral rod disposed in an orientation; and selectively rotating the proximal portion relative to the distal portion in a first plane of a body, and selectively rotating the pivoting member relative to the proximal portion in a second plane of the body, to the orientation to dispose the rod in the implant cavity.

### BRIEF DESCRIPTION OF THE DRAWINGS

The present disclosure will become more readily apparent from the specific description accompanied by the following drawings, in which:
FIG. 1 is a perspective view of one embodiment of a bone fastener of a system in accordance with the principles of the present disclosure, with parts separated;
FIG. 2 is a side view of the bone fastener shown in FIG. 1;
FIG. 3 is a cross section view of the bone fastener taken along lines A-A as shown in FIG. 2;
FIG. 4 is a break away, side view of the bone fastener shown in FIG. 1;
FIG. 5 is a side view of a component of the bone fastener shown in FIG. 1;
FIG. 6 is a side view of the component shown in FIG. 5;
FIG. 7 is a cross section view of the component taken along lines B-B as shown in FIG. 6;
FIG. 8 is an end view of the component shown in FIG. 5;
FIG. 9 is a side view of a component of the bone fastener shown in FIG. 1;
FIG. 10 is a side view of the component shown in FIG. 9;
FIG. 11 is a cross section view of the component taken along lines C-C as shown in FIG. 10;
FIG. 12 is an enlarged cross section view of the component taken at detail D as shown in FIG. 10;
FIG. 13 is an end view of the component shown in FIG. 9;
FIG. 14 is an end view of the component shown in FIG. 9;
FIG. 15 is a perspective view of a component of the bone fastener shown in FIG. 1;
FIG. 16 is a cross section view of the component taken along lines E-E as shown in FIG. 15;
FIG. 17 is a cross section view of the component taken along lines F-F as shown in FIG. 15;
FIG. 18 is an end view of a component of the bone fastener shown in FIG. 1;
FIG. 19 is a cross section view of the component taken along lines G-G as shown in FIG. 18;
FIG. 20 is a breakaway, cross section view of the bone fastener shown in FIG. 1;
FIG. 21 is a break away, cross section view of the bone fastener shown in FIG. 1;
FIG. 22 is a break away, cross section view of the bone fastener shown in FIG. 1;
FIG. 23 is side view of a system in accordance with the principles of the present disclosure disposed with vertebrae; and
FIG. 24 is a plan view of the system shown in FIG. 22 disposed with vertebrae.

Like reference numerals indicate similar parts throughout the figures.

### DETAILED DESCRIPTION

The exemplary embodiments of a surgical system and methods of use disclosed are discussed in terms of medical devices for the treatment of spinal disorders and more particularly, in terms of a spinal implant system including a bone fastener that provides stabilization while reducing stress on spinal elements. In one embodiment, the spinal implant system includes a transverse sagittal angulating and accommodating screw. The screw provides direct control of an implant.

In one embodiment, the spinal implant system allows sagittal accommodation to a spinal rod. It is envisioned that this configuration allows for sagittal manipulation once a spinal rod has been placed into a screw. It is further envisioned that the screw allows a head of the screw to pivot in a transverse plane of a body of a patient. It is contemplated that the screw may have a pivoting head combined with a pivoting saddle to allow sagittal accommodation to a spinal rod and sagittal manipulation once a spinal rod has been positioned within the head of the screw.

In one embodiment, the spinal implant system allows for transverse movement of a head of a screw while allowing a saddle to pivot in the sagittal plane. In one embodiment, the position of the screw and/or the head may lock after tightening the assembly of the system. It is contemplated that this configuration includes the saddle contacting the screw head and/or a gap disposed between the saddle and the screw head so that these components do not fully compress on each other, such as, the saddle locking against the head rather than the screw. In one embodiment, the bone fastener includes a head that pivots approximately in a range of 50 degrees in a transverse plane. It is contemplated that such range can be measured +/- 25 degrees from an axis. In one embodiment, the bone fastener includes a saddle that pivots approximately in a range of 26 degrees in a sagittal plane. It is contemplated that such range can be measured +/- 13 degrees from an axis.

In one embodiment, the bone fastener has a low profile to allow an implant, such as, for example, a vertebral rod to be disposed in close proximity to a bone or other tissue into which the bone fastener is fixed. It is contemplated that the bone fastener will provide approximately 0.10 to 0.75 millimeters (mm) of space between bone or other tissue into which the bone fastener is fixed and an implant disposed with the bone fastener.

It is envisioned that the present disclosure may be employed to treat spinal disorders such as, for example, degenerative disc disease, disc herniation, osteoporosis, spondylolisthesis, stenosis, scoliosis and other curvature abnormalities, kyphosis, tumor and fractures. It is contemplated that the present disclosure may be employed with other osteal and bone related applications, including those associated with diagnostics and therapeutics. It is further contemplated that the disclosed bone fasteners and methods may be alternatively employed in a surgical treatment with a patient in a prone or supine position, and/or employ various surgical approaches to the spine, including anterior, posterior, posterior mid-line, lateral, postero-lateral, and/or antero-lateral approaches, and in other body regions. The present disclosure may also be alternatively employed with procedures for treating the lumbar, cervical, thoracic and pelvic regions of a spinal column. The bone fasteners and methods of the present disclosure may also be used on animals, bone models and other non-living substrates, such as, for example, in training, testing and demonstration.

The present disclosure may be understood more readily by reference to the following detailed description of the disclosure taken in connection with the accompanying drawing figures, which form a part of this disclosure. It is to be understood that this disclosure is not limited to the specific devices, methods, conditions or parameters described and/or shown herein, and that the terminology used herein is for the purpose of describing particular embodiments by way of example only and is not intended to be limiting of the claimed disclosure. Also, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment. It is also understood that all spatial references, such as, for example, horizontal, vertical, top, upper, lower, bottom, left and right, are for illustrative purposes only and can be varied within the scope of the disclosure. For example, the references "upper" and "lower" are relative and used only in the context to the other, and are not necessarily "superior" and "inferior".

Further, as used in the specification and including the appended claims, "treating" or "treatment" of a disease or condition refers to performing a procedure that may include administering one or more drugs to a patient (human, normal or otherwise or other mammal), in an effort to alleviate signs or symptoms of the disease or condition. Alleviation can occur prior to signs or symptoms of the disease or condition appearing, as well as after their appearance. Thus, treating or treatment includes preventing or prevention of disease or undesirable condition (e.g., preventing the disease from occurring in a patient, who may be predisposed to the disease but has not yet been diagnosed as having it). In addition, treating or treatment does not require complete alleviation of signs or symptoms, does not require a cure, and specifically includes procedures that have only a marginal effect on the patient. Treatment can include inhibiting the disease, e.g., arresting its development, or relieving the disease, e.g., causing regression of the disease. For example, treatment can include reducing acute or chronic inflammation; alleviating pain and mitigating and inducing re-growth of new ligament, bone and other tissues; as an adjunct in surgery; and/or any repair procedure. Also, as used in the specification and including the appended claims, the term "tissue" includes soft tissue, ligaments, tendons, cartilage and/or bone unless specifically referred to otherwise.

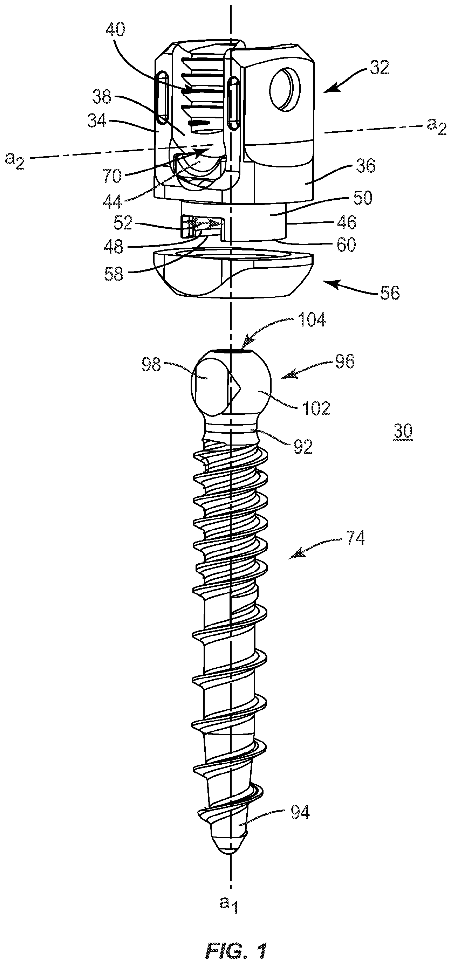

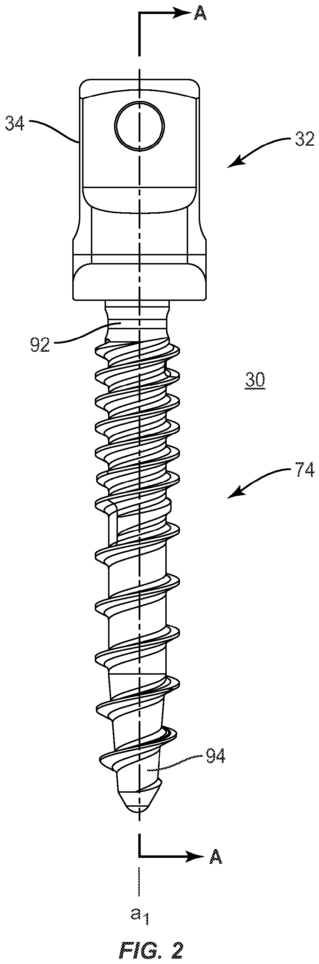

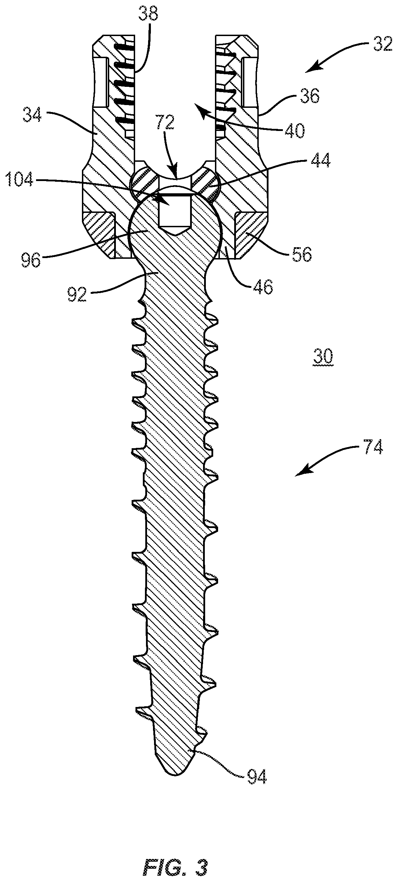

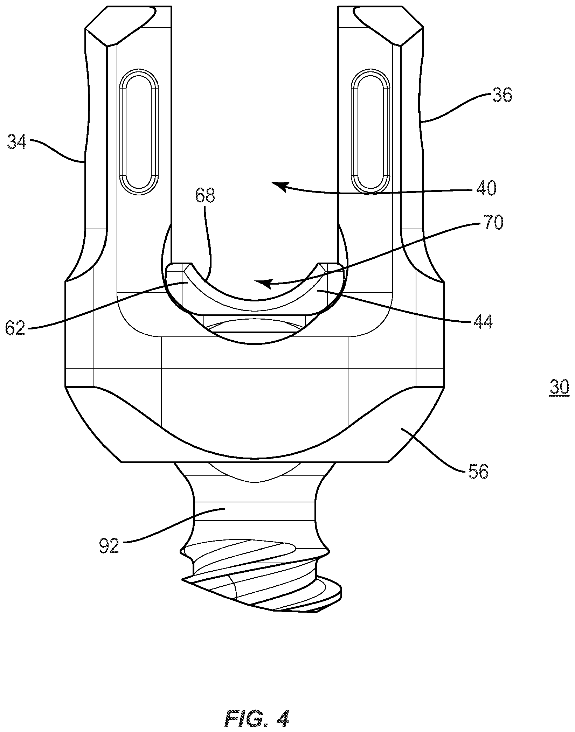

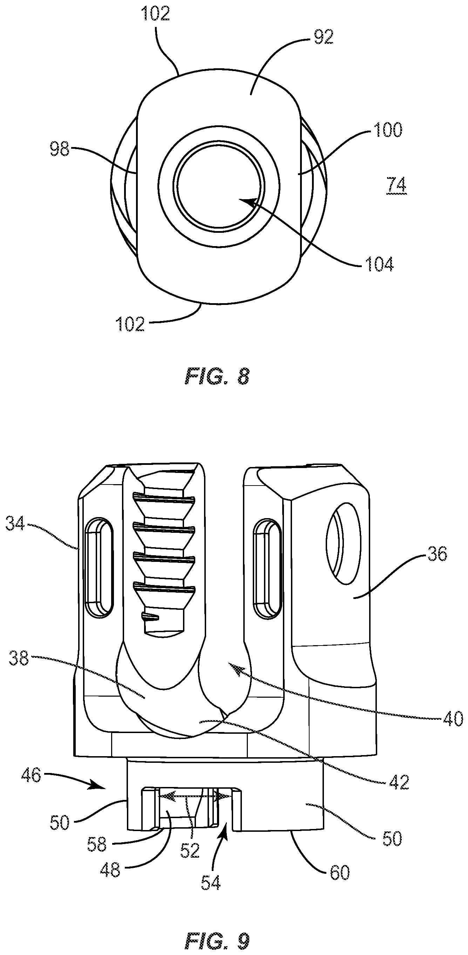

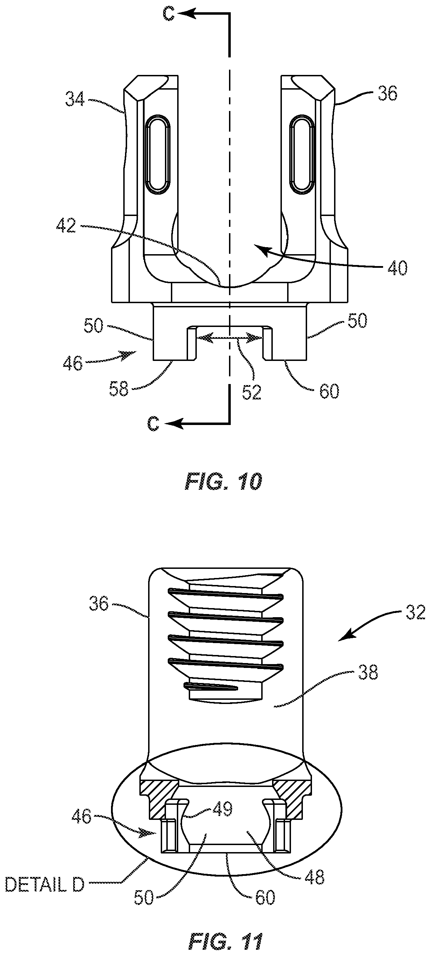

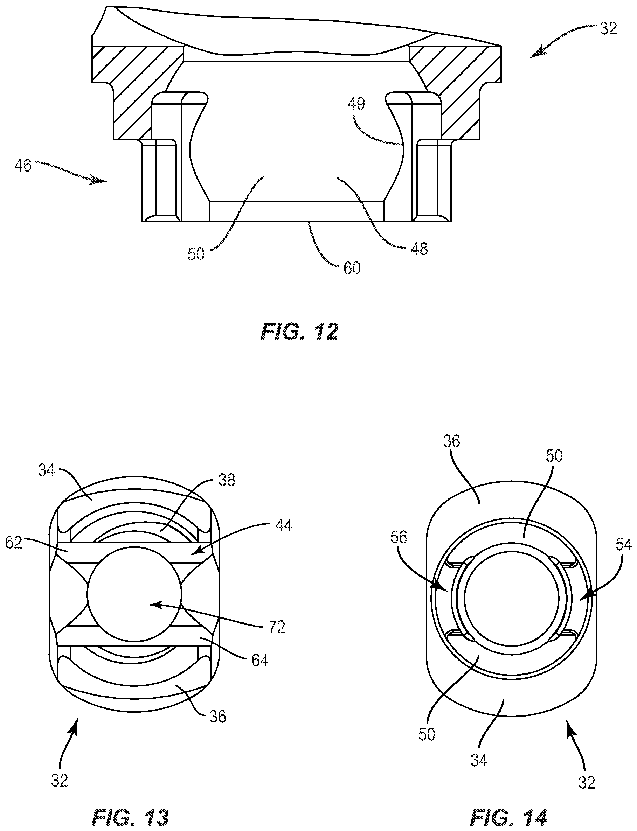

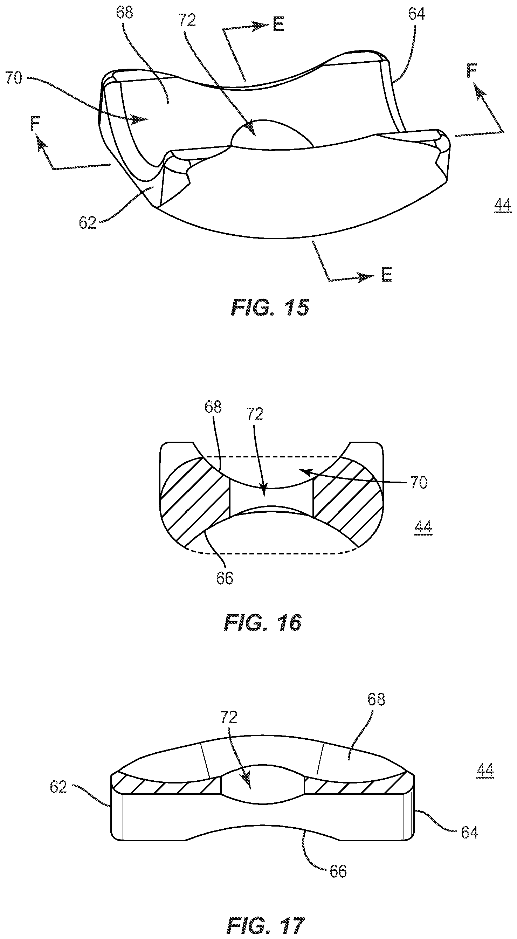

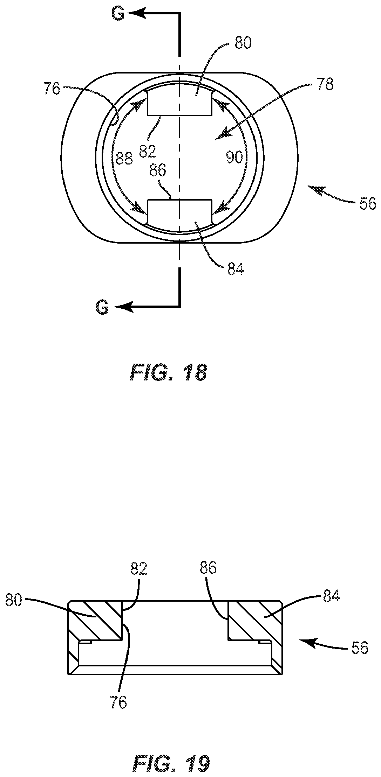

The following discussion includes a description of a spinal implant system including a bone fastener, related components and exemplary methods of employing the bone fastener in accordance with the principles of the present disclosure. Alternate embodiments are also disclosed. Reference will now be made in detail to the exemplary embodiments of the present disclosure, which are illustrated in the accompanying figures. Turning now to FIGS. 1-22, there is illustrated components of a spinal implant system including at least one bone fastener 30 in accordance with the principles of the present disclosure.

The components of the spinal implant system can be fabricated from biologically acceptable materials suitable for medical applications, including metals, synthetic polymers, ceramics and bone material and/or their composites, depending on the particular application and/or preference of a medical practitioner. For example, the components of bone fastener 30, individually or collectively, can be fabricated from materials such as stainless steel alloys, commercially pure titanium, titanium alloys, Grade 5 titanium, super-elastic titanium alloys, cobalt-chrome alloys, stainless steel alloys, superelastic metallic alloys (e.g., Nitinol, super elasto-plastic metals, such as GUM METAL® manufactured by Toyota Material Incorporated of Japan), ceramics and composites thereof such as calcium phosphate (e.g., SKELITE™ manufactured by Biologix Inc.), thermoplastics such as polyaryletherketone (PAEK) including polyetheretherketone (PEEK), polyetherketoneketone (PEKK) and polyetherketone (PEK), carbon-PEEK composites, PEEK-BaSO₄ polymeric rubbers, polyethylene terephthalate (PET), fabric, silicone, polyurethane, silicone-polyurethane copolymers, polymeric rubbers, polyolefin rubbers, hydrogels, semi-rigid and rigid materials, elastomers, rubbers, thermoplastic elastomers, thermoset elastomers, elastomeric composites, rigid polymers including polyphenylene, polyamide, polyimide, polyetherimide, polyethylene, epoxy, bone material including autograft, allograft, xenograft or transgenic cortical and/or corticocancellous bone, and tissue growth or differentiation factors, partially resorbable materials, such as, for example, composites of metals and calcium-based ceramics, composites of PEEK and calcium based ceramics, composites of PEEK with resorbable polymers, totally resorbable materials, such as, for example, calcium based ceramics such as calcium phosphate, tri-calcium phosphate (TCP), hydroxyapatite (HA)-TCP, calcium sulfate, or other resorbable polymers such as polyaetide, polyglycolide, polytyrosine carbonate, polycaroplaetohe and their combinations. Various components of the spinal implant system may have material composites, including the above materials, to achieve various desired characteristics such as strength, rigidity, elasticity, compliance, biomechanical performance, durability and radiolucency or imaging preference. The components of the spinal implant system, individually or collectively, may also be fabricated from a heterogeneous material such as a combination of two or more of the above-described materials. The components of the spinal implant system may be monolithically formed, integrally connected or include fastening elements and/or instruments, as described herein.

Bone fastener 30 comprises a proximal portion, such as, for example, a receiver 32 defining a first longitudinal axis a₁ and including spaced apart arms 34, 36 extending parallel to first longitudinal axis a₁ that include an inner surface 38 of receiver 32. It is contemplated that arm 34 and/or arm 36 may be disposed at alternate orientations, relative to first longitudinal axis a₁, such as, for example, transverse, perpendicular and/or other angular orientations such as acute or obtuse, co-axial and/or may be offset or staggered. Arms 34, 36 each include an arcuate outer surface. It is envisioned that the outer surfaces of arms 34, 36 may include a recess or cavity configured to receive an insertion tool, compression instrument and/or instruments for inserting and tensioning bone fastener 30.

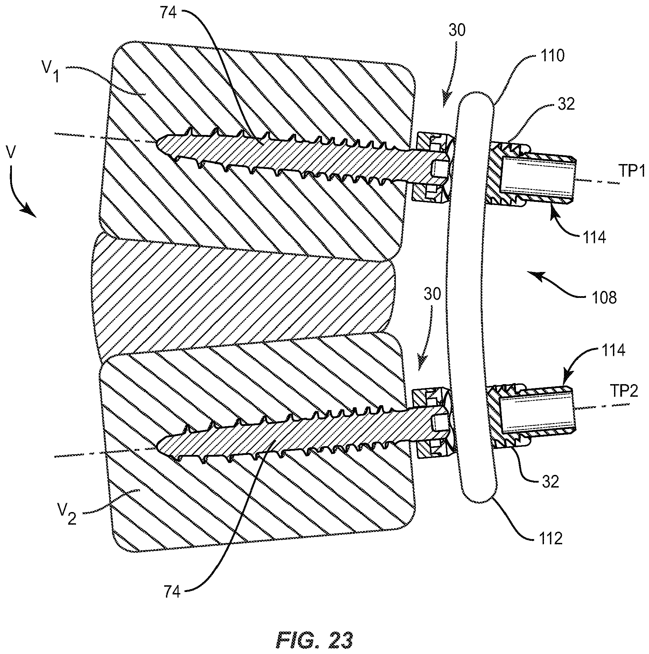

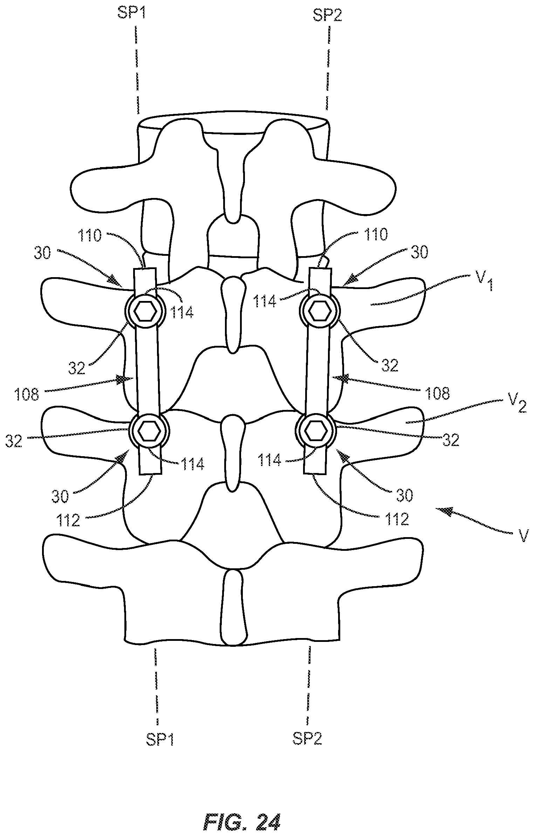

Inner surface 38 of receiver 32 defines a U-shaped cavity 40 extending between arms 34, 36. It is envisioned that all or only a portion of cavity 40 may have alternate cross section configurations, such as, for example, oval, oblong, triangular, square, polygonal, irregular, uniform, non-uniform, offset, staggered, and/or tapered. At least a portion of inner surface 38 is threaded and engageable with a coupling member, such as, for example, a setscrew. It is envisioned that inner surface 38 can include a thread form located adjacent arm 34 and a thread form located adjacent arm 36 each configured for engagement with a setscrew (FIGS. 23 and 24), as will be described. It is envisioned that inner surface 38 may be disposed with the setscrew in alternate fixation configurations, such as, for example, friction fit, pressure fit, locking protrusion/recess, locking keyway and/or adhesive. It is contemplated that all or only a portion of inner surface 38 may have alternate surface configurations to enhance fixation with the setscrew such as, for example, rough, arcuate, undulating, mesh, porous, semi-porous, dimpled and/or textured according to the requirements of a particular application.

Inner surface 38 of receiver 32 includes a concave surface 42 configured to receive at least a portion of a pivoting member, such as, for example, a saddle 44, described below, for engagement with receiver 32. Concave surface 42 extends distally and is recessed from inner surface 38. It is envisioned that concave surface 42 may be disposed in the center of inner surface 38 such that concave surface 42 is equidistant from arm 34 and arm 36. It is further envisioned that concave surface 42 may also be offset such that concave surface 42 is disposed closer to arm 34 than arm 36, or vice versa. It is contemplated that concave surface 42 can extend into inner surface 38 without extending through a bottom surface of receiver 32. Concave surface 42 is configured to receive a corresponding convexly curved portion of saddle 44. It is contemplated that all or only a portion of concave surface 42 may be variously configured and dimensioned, such as, for example, oval, oblong, square, rectangular, polygonal, irregular, uniform, non-uniform, offset, staggered, tapered, consistent or variable, depending on the requirements of a particular application.

Receiver 32 includes an extension, such as, for example, a distal collar 46 extending distally from a distal end of receiver 32 having an inner surface 48 defining a concavely curved circumferential wall 49 configured to engage at least a portion of a distal portion, such as, for example, a tissue penetrating shaft 74 such that receiver 32 is rotatable relative to shaft 74 in a plane of the body, as discussed below. Inner surface 48 includes at least one projection 50 defining a first lateral opening 52 and a second lateral opening 54 spaced apart from first lateral opening 52. Distal collar 46 is substantially circular and is configured for disposal in a component, such as, for example, a cap 56 of bone fastener 30 to engage receiver 32 with cap 56, as will be described. It is contemplated that all or only a portion of distal collar 46 may be variously configured and dimensioned, such as, for example, oval, oblong, square, rectangular, polygonal, irregular, uniform, non-uniform, offset, staggered, tapered, consistent or variable, depending on the requirements of a particular application. Projection(s) 50 has/have an arcuate shape and is/are engageable with shaft 74 in a configuration to retain shaft 74 with receiver 32, as will be described. It is envisioned that all or only a portion of projection(s) 50 may be variously configured and dimensioned, such as, for example, planar, concave, polygonal, irregular, uniform, non-uniform, staggered, tapered, consistent or variable, depending on the requirements of a particular application.

In one embodiment, bone fastener 30 comprises two projections 50, each engageable with shaft 74 to retain shaft 74 with receiver 32. It is envisioned that bone fastener 30 may comprise one or a plurality of projections 50, depending on the requirements of a particular application. A first projection 50 includes a first circumferential rim 58 extending between planar side surfaces and a second projection 50 includes a second circumferential rim 60 extending between planar side surfaces. Second circumferential rim 60 is spaced apart from first circumferential rim 58 a distance defined by a width of first lateral opening 52 and/or second lateral opening 54. The side surfaces of the first and second projections 50 extend parallel to first longitudinal axis a₁. It is contemplated that the side surfaces of the first and second projections 50 may be disposed at alternate orientations, relative to first longitudinal axis a₁, such as, for example, transverse, perpendicular and/or other angular orientations such as acute or obtuse, co-axial and/or may be offset or staggered. First and second circumferential rims 58, 60 are planar and extend transverse to first longitudinal axis a₁ such that the first and second projections 50 are substantially rectangular. It is envisioned that all or only a portion of the first projection 50 and/or the second projection 50 may be variously configured and dimensioned, such as, for example, oval, oblong, square, polygonal, irregular, uniform, non-uniform, offset, staggered, tapered, consistent or variable, depending on the requirements of a particular application. It is contemplated that first and second circumferential rims 58, 60 may be disposed at alternate orientations, relative to first longitudinal axis a₁, such as, for example, perpendicular and/or other angular orientations such as acute or obtuse, co-axial and/or may be offset or staggered. It is further contemplated that the side surfaces of the first and second projections 50 and first and second circumferential rims 58, 60 may be variously configured and dimensioned, such as, for example, convex, concave, polygonal, irregular, uniform, non-uniform, staggered, tapered, consistent or variable, depending on the requirements of a particular application.

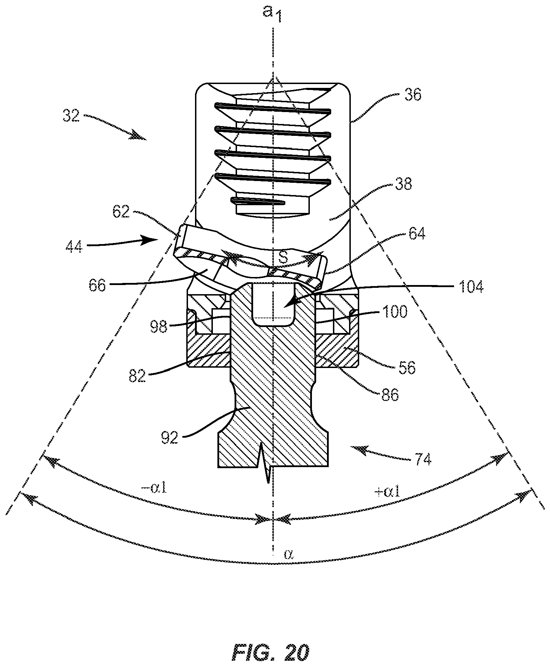

Saddle 44 extends between a first end 62 and a second end 64 and is disposed with concave surface 42 of receiver 32. Saddle 44 defines a first surface 66 that is curved between first and second ends 62, 64 and configured for slidable engagement with inner surface 38 of receiver 32 along arcuate path S (FIG. 20). First surface 66 is configured to engage at least a portion of shaft 74 such that receiver 32 is rotatable relative to shaft 74 in a plane of a body. Saddle 44 defines a second concave surface 68 that defines an implant cavity 70 with receiver 32. Implant cavity 70 defines a second axis a₂ transverse to first longitudinal axis a₁. Implant cavity 70 is configured to receive and movably support at least a portion of an implant, such as, for example, a vertebral rod such that the implant can translate axially relative to implant cavity 70 along second axis a₂ prior to fixation and is pivotable with saddle 44. It is contemplated that at least a portion of the implant may be disposed within implant cavity 70 for relative movement in orientations relative to second axis a₂, such as, for example, transverse, perpendicular and/or other angular orientations such as acute or obtuse, co-axial and/or may be offset or staggered. It is envisioned that implant cavity 70 may have alternate cross section configurations, such as, for example, oval, oblong, triangular, rectangular, square, polygonal, irregular, uniform, non-uniform, variable and/or tapered.

Saddle 44 includes a through opening 72 extending through first surface 66 and second concave surface 68 along first longitudinal axis a₁ configured for alignment with shaft 74, as will be described. Through opening 72 is substantially circular and defines a passageway through saddle 44 for a tool. It is envisioned that all or only a portion of through opening 72 may be variously configured and dimensioned, such as, for example, oval, oblong, triangular, square, rectangular, polygonal, irregular, uniform, non-uniform, offset, staggered, tapered, consistent or variable, depending on the requirements of a particular application.

It is envisioned that saddle 44 may be elastic and pliable in a configuration to react to forces applied and/or force changes, such as, for example, patient growth, trauma and degeneration, and/or component creep, deformation, damage and degeneration, to maintain the applied force transmitted from an implant positioned in implant cavity 70 substantially constant. It is contemplated that saddle 44 can facilitate maintenance of a holding force on an implant positioned in implant cavity 70 to remain the holding force relatively constant despite growth and changes to bone fastener 30.

Saddle 44 translates relative to receiver 32 along arcuate path S such that saddle 44 is selectively rotatable relative to receiver 32 in a plane, such as, for example, a sagittal plane of a body of a patient. Saddle 86 is rotatable about second axis a₂ through an angular range α (FIG. 20). Saddle 44 is pivotable along arcuate path S in slidable engagement with concave surface 42 through angular range α at +/- an angle α1 relative to axis a1. It is contemplated that angular range α may include a range of approximately 0 to 26 degrees. It is further contemplated that angle α1 may include a range of approximately +/- 13 degrees. It is contemplated that saddle 44 may be disposed with receiver 32 for relative movement in orientations relative to second axis a₂, such as, for example, transverse, perpendicular and/or other angular orientations such as acute or obtuse, co-axial and/or may be offset or staggered. It is further contemplated that saddle 44 may move relative to receiver 32 in alternate planes relative to a body, such as, for example, vertical, horizontal, diagonal, transverse, coronal and/or sagittal planes of a body.

Cap 56 is configured for fixed engagement with distal collar 46 of receiver 32 and includes an inner surface 76 that defines an inner cavity 78 configured for disposal of distal collar 46. Cap 56 further includes at least one planar surface, such as, for example, a first inwardly oriented tab 80 that includes a first planar surface 82 and a second inwardly oriented tab 84 that includes a second planar surface 86. First planar surface 82 extends between planar end surfaces of first inwardly oriented tab 80 and second planar surface 86 extends between planar end surfaces of second inwardly oriented tab 84. First planar face 82 is oriented in a first direction and second planar face 86 is oriented in a second direction, which is opposite to the first direction. It is envisioned that bone fastener 30 may comprise one or a plurality of tabs, depending on the requirements of a particular application. It is further envisioned that first planar surface 82, second planar surface 86, the side surfaces of first inwardly oriented tab 82 and/or the side surfaces of second inwardly oriented tab 84 may be variously configured and dimensioned, such as, for example, convex, concave, polygonal, irregular, uniform, non-uniform, staggered, tapered, consistent or variable, depending on the requirements of a particular application.

Cap 56 includes a first channel 88 extending between an end surface of first inwardly oriented tab 80 and an end surface of second inwardly oriented tab 84 configured for disposal of first projection 50 of distal collar 46 and a second channel 90 extending between an opposite end surface of first inwardly oriented tab 80 and an opposite end surface of second inwardly oriented tab 84 configured for disposal of second projection 50 of distal collar 46. First and second projections 50 are aligned with first and second channels 88, 90 and receiver 32 is advanced distally along longitudinal axis a₁ such that first and second projections 50 are disposed in first and second channels 88, 90 to engage cap 56 with receiver 32. As cap 56 engages receiver 32, the end surfaces of first inwardly oriented tab 82 engage the planar side surfaces of first projection 50 and the end surfaces of second inwardly oriented tab engage the planar side surfaces of second projection 50 such that rotation of receiver 32 relative to shaft 74 is prevented about first longitudinal axis a₁. Once cap 56 engages receiver 32, as described, cap 56 and receiver 32 may be permanently joined by welding the two components together using techniques, such as, for example, spot welding, cold welding or laser welding. It is envisioned that the planar side surfaces of first and second projections 50 and the end surfaces of first and second inwardly oriented tabs 80, 84 may be variously configured and dimensioned, such as, for example, planar, concave, polygonal, irregular, uniform, non-uniform, staggered, tapered, consistent or variable, depending on the requirements of a particular application. It is further envisioned that receiver 32 may be disposed with cap 56 in alternate fixation configurations, such as, for example, friction fit, pressure fit, locking protrusion/recess, locking keyway and/or adhesive.

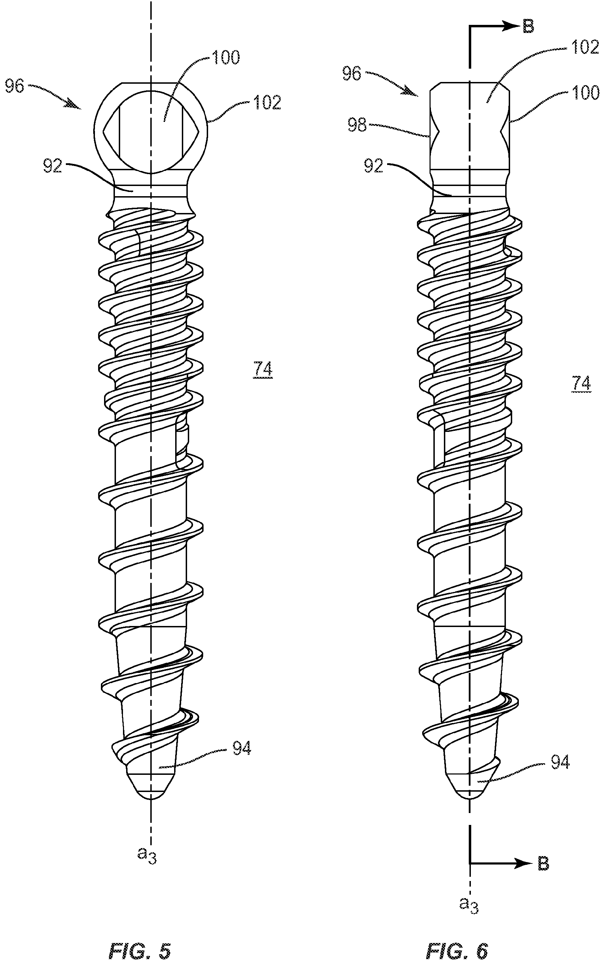

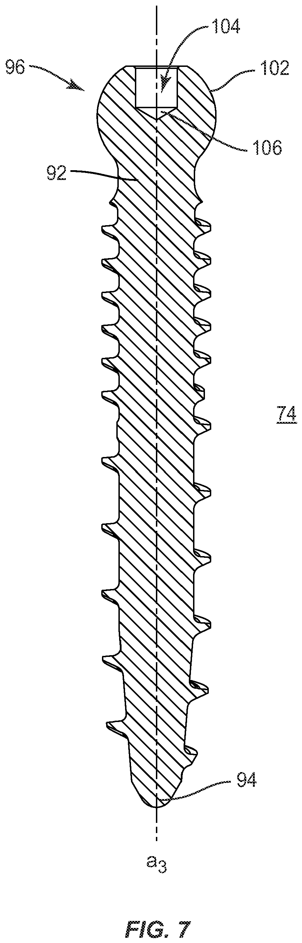

Shaft 74, shown in FIGS. 6 and 7, extends between a first end 92 and a second end 94 defining a third longitudinal axis a₃. Shaft 46 has a cylindrical cross section configuration that extends to a pointed distal tip. Shaft 74 includes an outer surface having an external threaded form. It is contemplated that the thread form on the outer surface of shaft 74 may include a single thread turn or a plurality of discrete threads. It is further contemplated that other engaging structures may be located on shaft 74, such as, for example, a nail configuration, barbs, expanding elements, raised elements and/or spikes to facilitate engagement of shaft 46 with tissue, such as, for example, vertebrae.

It is envisioned that all or only a portion of shaft 74 may have alternate cross section configurations, such as, for example, oval, oblong, triangular, square, polygonal, irregular, uniform, non-uniform, offset, staggered, undulating, arcuate, variable and/or tapered. It is contemplated that the outer surface of shaft 74 may include one or a plurality of openings. It is contemplated that all or only a portion of the outer surface of shaft 74 may have alternate surface configurations to enhance fixation with tissue such as, for example, rough, arcuate, undulating, mesh, porous, semi-porous, dimpled and/or textured according to the requirements of a particular application. It is envisioned that all or only a portion of shaft 74 may be disposed at alternate orientations, relative to third longitudinal axis a₃, such as, for example, transverse, perpendicular and/or other angular orientations such as acute or obtuse, co-axial and/or may be offset or staggered. It is further envisioned that all or only a portion of shaft 74 may be cannulated.

First end 92 of shaft 74 includes a head 96 engageable with first and second inwardly oriented tabs 80, 84 in a configuration to retain shaft 74 with receiver 32. Head 96 includes a first planar surface 98 oriented in a first direction configured to engage first planar surface 82 of first inwardly oriented tab 80. A second planar surface 100 is oriented in a second direction configured to engage second planar surface 86 of second inwardly oriented tab 84.

In one embodiment, head 96 is inserted with and passed through inner cavity 78 of cap 56 such that planar surfaces 98, 100 slide past, about and/or along planar surfaces 82, 86. Cap 56 and/or shaft 74 are advanced along third longitudinal axis a₃ for engagement and fixation with distal collar 46. Head 96 is received with the cavity defined by surface 48, via axial, rotational and/or angular alignment. Head 96 engages wall 49, and wall 49 and rims 58, 60 fix and/or retain head 96 with collar 46. It is contemplated that head 96 is fixed with wall 49 in various fixation configurations, such as, for example, snap fit, friction fit, pressure fit, clips and/or adhesive.

Upon assembly of shaft 74 with receiver 32, first and second planar surfaces 98, 100 of shaft 74 are aligned with first and second planar surfaces 82, 86 of cap 56. to engage first planar surface 82 with first planar surface 98 and second planar surface 86 with second planar surface 100 to prevent rotation of shaft 74 relative to receiver 32 and cap 56 about third longitudinal axis a₃. This configuration allows a tool to engage and rotate shaft 74, and simultaneously rotate receiver 32 due to the engagement of the planar surfaces. It is envisioned that first and second planar surfaces 98, 100 of shaft 74 and/or first and second planar surfaces 82, 86 of cap 56 may be variously configured and dimensioned, such as, for example, convex, concave, polygonal, irregular, uniform, non-uniform, staggered, tapered, consistent or variable, depending on the requirements of a particular application. It is further envisioned that shaft 74 may be retained with cap 56 in alternate fixation configurations, such as, for example, friction fit, pressure fit, locking protrusion/recess, locking keyway and/or adhesive.

Shaft has an arcuate surface 102 disposed between first and second planar surfaces 98, 100. Arcuate surface 102 is engageable and moveable relative to the concavely curved circumferential wall 49 of distal collar 46 and the curved first surface 66 of saddle 44 such that receiver 32 is capable of multi-axial positioning with respect to shaft 74 via rotation relative to shaft 74 in a plane of a body, such as, for example, a transverse plane of the body. It is contemplated that shaft 74 may be disposed with receiver 32 for relative movement in orientations relative to third longitudinal axis a₃, such as, for example, transverse, perpendicular and/or other angular orientations such as acute or obtuse, co-axial and/or may be offset or staggered. It is further contemplated that shaft 74 may move relative to receiver 32 in alternate planes relative to a body, such as, for example, vertical, horizontal, diagonal, coronal and/or sagittal planes of a body.

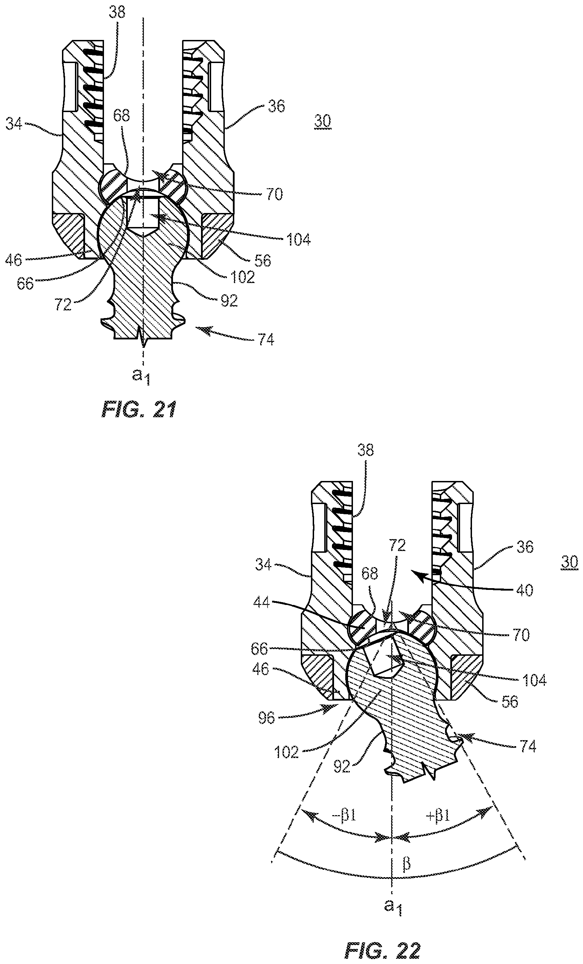

Head 96 of shaft 74 engages first surface 66 of saddle 44 proximate first end 62 to prevent movement of saddle 44 relative to shaft 74 in one direction and engages first surface 66 of saddle 44 proximate second end 64 to prevent movement of saddle 44 relative to shaft 74 in an opposite direction such that receiver 32 is selectively rotatable relative to shaft 74 within a transverse plane of a body through an angular range β (FIG. 22). Shaft 74 is pivotable through an angular range β at +/- an angle β1 relative to first longitudinal axis a₁. It is contemplated that angular range β may include a range of approximately 0 to 50 degrees. It is further contemplated that angle β1 may include a range of approximately +/- 25 degrees. It is contemplated that receiver 32 may be disposed with shaft 74 for relative movement in orientations relative to first longitudinal axis a₁, such as, for example, transverse, perpendicular and/or other angular orientations such as acute or obtuse, co-axial and/or may be offset or staggered. It is further contemplated that receiver 32 may move relative to shaft 74 in alternate planes relative to a body, such as, for example, vertical, horizontal, diagonal, coronal and/or sagittal planes of a body. It is envisioned that receiver 32 may be retained with shaft 74 in alternate fixation configurations, such as, for example, friction fit, pressure fit, locking protrusion/recess, locking keyway and/or adhesive.

First end 92 of shaft 74 defines a tool socket 104 configured for alignment with through opening 72 in saddle 44 such that through opening 72 and tool socket 104 are aligned with one another when cap 56 is engaged with receiver 32 and shaft 74 to define a passageway. An alignment tool may be advanced distally along first longitudinal axis a₁ such that the tool is inserted through cavity 40 of receiver 32 and through opening 72 of saddle 44 and into tool socket 104 to align receiver 32 and cap 56 with shaft 74. Tool socket 104 is substantially cylindrical. In one embodiment, tool socket 104 includes a tapered portion 106 (FIG. 7) having a first diameter at a proximal end and a second, reduced diameter at a distal end thereof. It is envisioned that all or only a portion of tool socket 104 may be variously configured and dimensioned, such as, for example, oval, oblong, square, rectangular, polygonal, irregular, uniform, non-uniform, offset, staggered, tapered, consistent or variable, depending on the requirements of a particular application.

In assembly, operation and use, a spinal implant system including bone fastener 30, similar to that described above, is employed with a surgical procedure for treatment of a spinal disorder affecting a section of a spine of a patient, as discussed herein. In particular, the spinal implant system is employed with a surgical procedure for treatment of a condition or injury of an affected section of the spine including vertebrae V, as shown in FIGS. 23 and 24. It is contemplated that the spinal implant system including bone fastener 30 is attached to vertebrae V for a surgical arthrodesis procedure, such as fusion, and/or dynamic stabilization application of the affected section of the spine to facilitate healing and therapeutic treatment.

In use, to treat the affected section of the spine, a medical practitioner obtains access to a surgical site including vertebra V in any appropriate manner, such as through incision and retraction of tissues. It is envisioned that the spinal implant system including bone fastener 30 may be used in any existing surgical method or technique including open surgery, mini-open surgery, minimally invasive surgery and percutaneous surgical implantation, whereby the vertebrae V is accessed through a micro-incision, or sleeve that provides a protected passageway to the area. Once access to the surgical site is obtained, the particular surgical procedure is performed for treating the spinal disorder. Bone fastener 30 is then employed to augment the surgical treatment. The spinal implant system including bone fastener 30 and a vertebral rod 108 can be delivered or implanted as a pre-assembled device or can be assembled in situ. The spinal implant system may be completely or partially revised, removed or replaced.

Pilot holes are made in vertebrae V₁ and V₂ for receiving shafts 74 of bone fasteners 30. Shafts 74 of first and second bone fasteners 30 are inserted or otherwise connected to vertebrae V₁ and V₂ according to the particular requirements of the surgical treatment. A pair of bone fasteners 30 are configured to attach upper sections 110 of rods 108 to vertebra V₁ and a pair of bone fasteners 30 are configured to attach lower sections 112 of rods 108 to adjacent vertebra V₂.

With shafts 74 connected to vertebrae V₁ and V₂, bone fasteners 30 are moveable between a first configuration and a second configuration. In the first configuration, each receiver 32 is attached with a shaft 74 such that receiver 32 is selectively and freely rotatable relative to shaft 74 within transverse planes TP1 and TP2 (FIG. 23), respectively, of vertebrae V. Saddle 44 is selectively and freely translatable relative to receiver 32 in sagittal planes SP1 and SP2 (FIG. 24), between a first moveable limit defined by engagement of first surface 66 of saddle 44 proximate first end 62 with arcuate surface 102 of shaft 74 and a second moveable limit defined by engagement of first surface 66 of saddle 44 proximate second end 64 with arcuate surface 102 (FIG. 20).

According to the orientation and position of sections 110, 112 of each rod 108, bone fasteners 30 are independently and selectively moved to a second configuration such that each implant cavity 70 of receiver 32 is selectively rotatable relative to shaft 74 within transverse planes TP1 and TP2. Implant cavity 70 is relatively rotatable such that receiver 32 rotates through an angular range β (FIG. 22) relative to axis a₁. Saddle 44 translates relative to receiver 32 along path S and is rotatable about first longitudinal axis a₁ through an angular range α (FIG. 20) in sagittal planes SP1 and SP2 to receive, engage and accommodate the orientation and position of sections 110, 112. Sections 110, 112 may also engage the individual saddles 44 to cause translation of a saddle 44 along path S. This configuration allows orientation of implant cavity 70 to receive each of sections 110, 112 such that receivers 32 can capture rods 108.

In the second configuration, setscrews 114 are torqued and threaded with each receiver 32 to securely attach rods 108 with vertebrae V₁, V₂. Each setscrew 114 is threaded into the threaded portion of inner surface 38 of receiver 32 such that setscrew 112 engages rod 108. As setscrew 114 is threaded into receiver 32, setscrew 114 applies a force to rod 108 disposed implant cavity 70. This force is transmitted through rod 108 such that rod 108 engages second concave surface 68 of saddle 44 causing at least a portion of inner surface 66 of saddle 44 to engage head 96 of shaft 74. This configuration fixes bone fastener 30 in an orientation to prevent receiver 32 from moving relative to shaft 74 such that bone fastener 30 may receive and accommodate the orientation and position of sections 110, 112.

Bone fastener 30 may be employed as a bone screw, pedicle screw or multi-axial screw used in spinal surgery. In one embodiment, the spinal implant system includes an agent, which may be disposed, packed or layered within, on or about the surfaces of bone fastener 30. It is envisioned that the agent may include bone growth promoting material, such as, for example, bone graft to enhance fixation of the fixation elements with vertebrae.

It is contemplated that the agent may include therapeutic polynucleotides or polypeptides. It is further contemplated that the agent may include biocompatible materials, such as, for example, biocompatible metals and/or rigid polymers, such as, titanium elements, metal powders of titanium or titanium compositions, sterile bone materials, such as allograft or xenograft materials, synthetic bone materials such as coral and calcium compositions, such as HA, calcium phosphate and calcium sulfite, biologically active agents, for example, gradual release compositions such as by blending in a bioresorbable polymer that releases the biologically active agent or agents in an appropriate time dependent fashion as the polymer degrades within the patient. Suitable biologically active agents include, for example, BMP, Growth and Differentiation Factors proteins (GDF) and cytokines. The components of the spinal implant system can be made of radiolucent materials such as polymers. Radiomarkers may be included for identification under x-ray, fluoroscopy, CT or other imaging techniques. It is envisioned that the agent may include one or a plurality of therapeutic agents and/or pharmacological agents for release, including sustained release, to treat, for example, pain, inflammation and degeneration.

It is envisioned that the use of microsurgical and image guided technologies may be employed to access, view and repair spinal deterioration or damage, with the aid of the spinal implant system. Upon completion of a procedure employing the spinal implant system described above, the surgical instruments and assemblies are removed and the incision is closed.

It will be understood that various modifications may be made to the embodiments disclosed herein. Therefore, the above description should not be construed as limiting, but merely as exemplification of the various embodiments.

## Claims

1. A bone fastener (30) comprising:
a receiver (32) including an inner surface (38) and an outer surface having an extension (46) that includes an inner surface (48) defining at least one projection (50);
a saddle (44) being pivotable relative to the inner surface (38) of the receiver (32), the saddle (44) including a first surface (64) that defines an implant cavity (70) with the inner surface (38) of the receiver (32) and a second surface (66);
a cap (56) engageable with the receiver (32) and including an inner surface (76) that defines an inner cavity (78) configured for disposal of the extension (46), the cap (56) further including at least one planar surface (82, 86); and
a shaft (74) defining a longitudinal axis and having a first end (92) and a second end (94) configured to penetrate tissue, the first end (92) including at least one planar surface (98, 100) configured to engage the at least one planar surface (82,86) of the cap (56),
wherein the receiver (32) is rotatable relative to the shaft (74) in a first plane (TP1, TP2) of a body and the saddle (44) is rotatable relative to the receiver (32) in a second plane (SP1, SP2) of the body,
**characterized in that** the inner surface (48) of the extension (46) includes at least one circumferential projection (49) engageable with the first end (92) in a configuration to retain the shaft (74) with the receiver (32), wherein in an assembled state of the bone fastener (30) the first end (92) of the shaft (74) is engaged with the at least one circumferential projection (49) and is in contact thereto.

2. A bone fastener (30) as recited in claim 1, wherein the inner surface (48) of the extension (46) includes at least one projection (50) engageable with the first end (92) in a configuration to retain the shaft (74) with the receiver (32).

3. A bone fastener (30) as recited in either claim 1 or claim 2, wherein the inner surface (38) of the receiver (32) includes a first projection (50) and a second projection (50) spaced apart from the first projection (50), the projections (50, 50) being engageable with the first end (92) in a configuration to retain the shaft (74) with the receiver (32).

4. A bone fastener (30) as recited in any of claims 1-3, wherein the cap (56) includes at least one inwardly oriented tab (80, 84) that includes the at least one planar surface (82, 86) of the cap (56).

5. A bone fastener (30) as recited in any of claims 1-4, wherein the at least one planar surface (82, 86) of the cap (56) includes a first planar surface (82) oriented in a first direction and a second planar surface (86) oriented in a second direction, which is opposite to the first direction.

6. A bone fastener (30) as recited in any of claims 1-5, wherein the cap (56) includes a first inwardly oriented tab (80) that includes a first planar surface (82) of the cap (56) and a second inwardly oriented tab (84) that includes a second planar surface (86) of the cap (56).

7. A bone fastener (30) as recited in any of claims 1-6, wherein the at least one planar surface (98, 100) of the first end (92) includes a first planar surface (98) oriented in a first direction and a second planar surface (100) oriented in a second direction, which is opposite to the first direction.

8. A bone fastener (30) as recited in any of claims 1-7, wherein the first end (92) includes an arcuate surface (102) engageable and moveable relative to the receiver (32).

9. A bone fastener (30) as recited in any of claims 1-8, wherein the first end (92) includes an arcuate surface (102) engageable and moveable relative to the receiver (102) and the saddle (44).

10. A bone fastener (30) as recited in any of claims 1-9, wherein the at least one planar surface (98, 100) of the first end (92) includes a first planar surface (98) and a second planar surface (100), and the at least one planar surface (82, 86) of the cap (56) includes a first planar surface (82) and a second planar surface (86), the first planar surface (98) of the first end (92) being engageable with the first planar surface (82) of the cap (56) and the second planar surface (100) of the first end (92) being engageable with the second planar surface (86) of the cap (56) in a configuration to prevent rotation of the cap (56) relative to the shaft (74) about the longitudinal axis.

11. A bone fastener (30) as recited in claim 10, wherein the cap (56) is fixed with the receiver (32) such that rotation of the receiver (32) relative to the shaft (74) is prevented about the longitudinal axis.

12. A bone fastener (30) as recited in any of claims 1-11, wherein the first end (92) includes a first planar surface (98), a second planar surface (100) and an arcuate surface (102) disposed therebetween, the arcuate surface (102) being engageable and moveable relative to the receiver (32).

## Patentansprüche

1. Eine Knochenbefestigungsvorrichtung (30), aufweisend:
eine Aufnahme (32), aufweisend eine Innenfläche (38) und eine Außenfläche mit einer Verlängerung (46), die eine Innenfläche (48) aufweist, die mindestens einen Vorsprung (50) definiert,
einen Sattel (44), der relativ zu der Innenfläche (38) der Aufnahme (32) drehbar ist, wobei der Sattel (44) eine erste Fläche (64), die mit der Innenfläche (38) der Aufnahme (32) einen Implantathohlraum (70) definiert, und eine zweite Fläche (66) aufweist,
eine Kappe (56), die mit der Aufnahme (32) in Eingriff bringbar ist und eine Innenfläche (76) aufweist, die einen inneren Hohlraum (78) definiert, der zum Anordnen der Verlängerung (46) eingerichtet ist, wobei die Kappe (56) ferner mindestens eine planare Fläche (82, 86) aufweist, und
einen Schaft (74), der eine Längsachse definiert und ein erstes Ende (92) und ein zweites Ende (94), das zum Eindringen in Gewebe eingerichtet ist, aufweist, wobei das erste Ende (92) mindestens eine planare Fläche (98, 100) aufweist, die konfiguriert ist, um mit der mindestens einen planaren Fläche (82, 86) der Kappe (56) in Eingriff zu sein,
wobei die Aufnahme (32) relativ zu dem Schaft (74) in einer ersten Ebene (TP1, TP2) eines Körpers drehbar ist und der Sattel (44) relativ zu der Aufnahme (32) in einer zweiten Ebene (SP1, SP2) des Körpers drehbar ist,
**dadurch gekennzeichnet, dass** die Innenfläche (48) der Verlängerung (46) mindestens einen Umfangsvorsprung (49) aufweist, der mit dem ersten Ende (92) in einer Konfiguration in Eingriff bringbar ist, um den Schaft (74) mit der Aufnahme (32) zu halten, wobei in einem Montagezustand der Knochenbefestigungsvorrichtung (30) das erste Ende (92) des Schafts (74) mit dem mindestens einen Umfangsvorsprung (49) in Eingriff ist und damit in Kontakt ist.

2. Eine Knochenbefestigungsvorrichtung (30) gemäß Anspruch 1, wobei die Innenfläche (48) der Verlängerung (46) mindestens einen Vorsprung (50) aufweist, der mit dem ersten Ende (92) in einer Konfiguration in Eingriff bringbar ist, um den Schaft (74) mit der Aufnahme (32) zu halten.

3. Eine Knochenbefestigungsvorrichtung (30) gemäß entweder Anspruch 1 oder Anspruch 2, wobei die Innenfläche (38) der Aufnahme (32) einen ersten Vorsprung (50) und einen zweiten Vorsprung (50) aufweist, der im Abstand von dem ersten Vorsprung (50) ist, wobei die Vorsprünge (50, 50) mit dem ersten Ende (92) in einer Konfiguration in Eingriff bringbar sind, um den Schaft (74) mit der Aufnahme (32) zu halten.

4. Eine Knochenbefestigungsvorrichtung (30) gemäß irgendeinem der Ansprüche 1 bis 3, wobei die Kappe (56) mindestens eine nach innen gerichtete Lasche (80, 84) aufweist, die die mindestens eine planare Fläche (82, 86) der Kappe (56) aufweist.

5. Eine Knochenbefestigungsvorrichtung (30) gemäß irgendeinem der Ansprüche 1 bis 4, wobei die mindestens eine planare Fläche (82, 86) der Kappe (56) eine erste planare Fläche (82), die in einer ersten Richtung ausgerichtet ist, und eine zweite planare Fläche (86) aufweist, die in einer zweiten Richtung ausgerichtet ist, die entgegengesetzt zu der ersten Richtung ist.

6. Eine Knochenbefestigungsvorrichtung (30) gemäß irgendeinem der Ansprüche 1 bis 5, wobei die Kappe (56) eine erste nach innen ausgerichtete Lasche (80), die eine erste planare Fläche (82) der Kappe (56) aufweist, und eine zweite nach innen ausgerichtete Lasche (84) aufweist, die eine zweite planare Fläche (86) der Kappe (56) aufweist.

7. Eine Knochenbefestigungsvorrichtung (30) gemäß irgendeinem der Ansprüche 1 bis 6, wobei die mindestens eine planare Fläche (98, 100) des ersten Endes (92) eine erste planare Fläche (98), die in einer ersten Richtung ausgerichtet ist, und eine zweite planare Fläche (100) aufweist, die in einer zweiten Richtung ausgerichtet ist, die entgegengesetzt zu der ersten Richtung ist.

8. Eine Knochenbefestigungsvorrichtung (30) gemäß irgendeinem der Ansprüche 1 bis 7, wobei das erste Ende (92) eine bogenförmige Fläche (102) aufweist, die relativ zu der Aufnahme (32) in Eingriff bringbar und bewegbar ist.

9. Eine Knochenbefestigungsvorrichtung (30) gemäß irgendeinem der Ansprüche 1 bis 8, wobei das erste Ende (92) eine bogenförmige Fläche (102) aufweist, die relativ zu der Aufnahme (102) und dem Sattel (44) in Eingriff bringbar und bewegbar ist.

10. Eine Knochenbefestigungsvorrichtung (30) gemäß irgendeinem der Ansprüche 1 bis 9, wobei die mindestens eine planare Fläche (98, 100) des ersten Endes (92) eine erste planare Fläche (98) und eine zweite planare Fläche (100) aufweist, und die mindestens eine planare Fläche (82, 86) der Kappe (56) eine erste planare Fläche (82) und eine zweite planare Fläche (86) aufweist, wobei die erste planare Fläche (98) des ersten Endes (92) mit der ersten planaren Fläche (82) der Kappe (56) in Eingriff bringbar ist und die zweite planare Fläche (100) des ersten Endes (92) mit der zweiten planaren Fläche (86) der Kappe (56) in Eingriff bringbar ist, in einer Konfiguration, um ein Drehen der Kappe (56) relativ zu dem Schaft (74) um die Längsachse zu verhindern.

11. Eine Knochenbefestigungsvorrichtung (30) gemäß Anspruch 10, wobei die Kappe (56) mit der Aufnahme (32) fixiert ist, so dass ein Drehen der Aufnahme (32) relativ zu dem Schaft (74) um die Längsachse verhindert wird.

12. Eine Knochenbefestigungsvorrichtung (30) gemäß irgendeinem der Ansprüche 1 bis 11, wobei das erste Ende (92) eine erste planare Fläche (98), eine zweite planare Fläche (100) und eine dazwischen angeordnete bogenförmige Fläche (102) aufweist, wobei die bogenförmige Fläche (102) relativ zu der Aufnahme (32) in Eingriff bringbar und bewegbar ist.

## Revendications

1. Une fixation d'os (30), comportant :
un récepteur (32) comprenant une surface intérieure (38) et une surface extérieure présentant une extension (46) qui comprend une surface intérieure (48) définissant au moins une saillie (50),
une selle (44) étant pivotable relativement à la surface intérieure (38) du récepteur (32), la selle (44) comprenant une première surface (64) qui définit une cavité d'implant (70) avec la surface intérieure (38) du récepteur (32) et une deuxième surface (66),
un capuchon (56) pouvant se mettre en prise avec le récepteur (32) et comprenant une surface intérieure (76) qui définit une cavité intérieure (78) configurée pour l'agencement de l'extension (46), le capuchon (56) comprenant en outre au moins une surface plane (82, 86), et
une tige (74) définissant un axe longitudinal et présentant une première extrémité (92) et une deuxième extrémité (94) configurée pour pénétrer dans un tissu, la première extrémité (92) comprenant au moins une surface plane (98, 100) configurée pour se mettre en prise avec l'au moins une surface plane (82, 86) du capuchon (56),
dans laquelle le récepteur (32) est rotatif par rapport à la tige (74) dans un premier plan (TP1, TP2) d'un corps et la selle (44) est rotative par rapport au récepteur (32) dans un deuxième plan (SP1, SP2) du corps,
**caractérisée en ce que** la surface intérieure (48) de l'extension (46) comprend au moins une saillie circonférentielle (49) pouvant se mettre en prise avec la première extrémité (92) dans une configuration pour retenir la tige (74) avec le récepteur (32), où, dans un état monté de la fixation d'os (30), la première extrémité (92) de la tige (74) est en prise avec l'au moins une saillie circonférentielle (49) et est en contact avec celle-ci.

2. Une fixation d'os (30) selon la revendication 1, dans laquelle la surface intérieure (48) de l'extension (46) comprend au moins une saillie (50) pouvant se mettre en prise avec la première extrémité (92) dans une configuration pour retenir la tige (74) avec le récepteur (32).

3. Une fixation d'os (30) selon la revendication 1 ou la revendication 2, dans laquelle la surface intérieure (38) du récepteur (32) comprend une première saillie (50) et une deuxième saillie (50) espacée de la première saillie (50), les saillies (50, 50) pouvant se mettre en prise avec la première extrémité (92) dans une configuration pour retenir la tige (74) avec le récepteur (32).

4. Une fixation d'os (30) selon l'une quelconque des revendications 1 à 3, dans laquelle le capuchon (56) comprend au moins une languette (80, 84) orientée vers l'intérieur qui comprend l'au moins une surface plane (82, 86) du capuchon (56) .

5. Une fixation d'os (30) selon l'une quelconque des revendications 1 à 4, dans laquelle l'au moins une surface plane (82, 86) du capuchon (56) comprend une première surface plane (82) orientée dans une première direction et une deuxième surface plane (86) orientée dans une deuxième direction qui est opposée à la première direction.

6. Une fixation d'os (30) selon l'une quelconque des revendications 1 à 5, dans laquelle le capuchon (56) comprend une première languette (80) orientée vers l'intérieur qui comprend une première surface plane (82) du capuchon (56) et une deuxième languette (84) orientée vers l'intérieur qui comprend une deuxième surface plane (86) du capuchon (56).

7. Une fixation d'os (30) selon l'une quelconque des revendications 1 à 6, dans laquelle l'au moins une surface plane (98, 100) de la première extrémité (92) comprend une première surface plane (98) orientée dans une première direction et une deuxième surface plane (100) orientée dans une deuxième direction qui est opposée à la première direction.

8. Une fixation d'os (30) selon l'une quelconque des revendications 1 à 7, dans laquelle la première extrémité (92) comprend une surface arquée (102) pouvant se mettre en prise et étant mobile par rapport au récepteur (32).

9. Une fixation d'os (30) selon l'une quelconque des revendications 1 à 8, dans laquelle la première extrémité (92) comprend une surface arquée (102) pouvant se mettre en prise et étant mobile par rapport au récepteur (102) et à la selle (44).

10. Une fixation d'os (30) selon l'une quelconque des revendications 1 à 9, dans laquelle l'au moins une surface plane (98, 100) de la première extrémité (92) comprend une première surface plane (98) et une deuxième surface plane (100), et l'au moins une surface plane (82, 86) du capuchon (56) comprend une première surface plane (82) et une deuxième surface plane (86), la première surface plane (98) de la première extrémité (92) pouvant se mettre en prise avec la première surface plane (82) du capuchon (56) et la deuxième surface plane (100) de la première extrémité (92) pouvant se mettre en prise avec la deuxième surface plane (86) du capuchon (56) dans une configuration pour empêcher une rotation du capuchon (56) par rapport à la tige (74) autour de l'axe longitudinal.

11. Une fixation d'os (30) selon la revendication 10, dans laquelle le capuchon (56) est fixé avec le récepteur (32) de sorte qu'une rotation du récepteur (32) par rapport á la tige (74) est empêchée autour de l'axe longitudinal.

12. Une fixation d'os (30) selon l'une quelconque des revendications 1 à 11, dans laquelle la première extrémité (92) comprend une première surface plane (98), une deuxième surface plane (100) et une surface arquée (102) disposée entre celles-ci, la surface arquée (102) pouvant se mettre en prise et étant mobile par rapport au récepteur (32).
